# EUROPEAN PATENT APPLICATION

(11) **EP 4 830 738 A1**
(43) Date of publication of application: **28.10.2026**
(21) Application number: 26173950.2
(22) Date of filing: 22.04.2026
(51) Int. Cl.: A61L 17/00, A61L 17/14

(54) **COATED EPTFE SUTURE**

(30) Priority: 22.04.2025 US 202563792613 P; 11.03.2026 US 202619563282
(71) Applicant: Ethicon, Inc., Raritan, NJ 08869 (US)
(72) Inventor: OU, Duan Li, Raritan, 08869 (US)
(74) Representative: Carpmaels & Ransford LLP

(57) **Abstract**

A method for coating an expanded polytetrafluoroethylene (ePTFE) suture includes applying a chemical etchant to a surface of the ePTFE suture, the chemical etchant comprising an alkali-based etchant to remove fluorine from the surface of the ePTFE suture to produce an etched ePTFE suture; and applying a coating to the etched ePTFE suture to produce a coated ePTFE suture, the coating comprising a cross linkable silicone polymer, the coating comprising an antimicrobial agent selected from the group including of triclosan, chlorhexidine, polyhexamethylene biguanide (PHMB), octenidine, and silver.

## Description

### Priority Claim

The present disclosure claims priority to U.S. Provisional Patent Application Serial No. 63/792,613 filed April 22, 2025; the disclosure of which is incorporated herewith by reference.

### Background

Surgical sutures are used to close wounds and surgical incisions, and to repair damaged or severed muscles, vessels, and tissue. Typically, the suture is attached at one end to a needle, and the needle is drawn through tissue to form one or more loops holding the tissue together. The suture is subsequently tied off using one or more knots so that the tissue will remain drawn together. Sutures can be applied manually (i.e., by the hand of the surgeon) or by a suturing device, e.g., endoscopically.

Sutures can be formed from a variety of natural and synthetic materials including polytetrafluoroethylene (PTFE) and expanded PTFE (ePTFE). Sutures formed from PTFE are commonly used for their strength and biocompatibility. Sutures formed from ePTFE are very strong, soft, and smooth, making ePTFE sutures ideal for a number of surgical procedures including, e.g., dental bone grafting, implant procedures, and chordal replacement, where a soft monofilament suture is desirable. These properties of ePTFE also make ePTFE sutures easy to knot. However, there exists a risk of knots unraveling due to the slippery nature of ePTFE. Additionally, the porous nature of ePTFE can invite the growth of bacteria and/or other pathogenic microorganisms.

### Summary

The present disclosure relates to a method for coating an expanded polytetrafluoroethylene (ePTFE) suture which includes applying a chemical etchant to a surface of the ePTFE suture, the chemical etchant comprising an alkali-based etchant to remove fluorine from the surface of the ePTFE suture to produce an etched ePTFE suture; and applying a coating to the etched ePTFE suture to produce a coated ePTFE suture, the coating comprising a cross linkable silicone polymer, the coating comprising an antimicrobial agent selected from the group including of triclosan, chlorhexidine, polyhexamethylene biguanide (PHMB), octenidine, and silver.

In an embodiment, applying the chemical etchant comprises immersing the PTFE suture in a container including the alkali-based etchant for a set duration at a set temperature until the surface of the PTFE suture changes from a deep dark blue color to a transparent light brown color.

In an embodiment, the set duration comprises two minutes and the set temperature comprises 50°C, wherein applying the chemical etchant further comprises shaking the container periodically.

In an embodiment, applying the coating comprises dipping the etched ePTFE suture into a container including the coating; or brushing, rolling, or spraying the coating onto the surface of the etched ePTFE suture.

In an embodiment, the method further comprises curing the coating by a convection oven with curing parameters including a set duration at a set temperature, or flash curing the coating.

In an embodiment, first curing parameters comprise 5 seconds at 150°C and second curing parameters comprise 120 seconds at 90°C.

In an embodiment, removing fluorine from the surface of the ePTFE suture reduces a lubricity of the etched ePTFE suture relative to the ePTFE suture, wherein a lubricity of the coated ePTFE suture is reduced relative to the ePTFE suture.

In an embodiment, the coated ePTFE suture comprises a controlled release of the antimicrobial agent over a duration comprising at least 3 days.

In an embodiment, the alkali-based etchant comprises sodium naphthalene or sodium ammonia.

In an embodiment, curing the coating produces a cross-linked silicone matrix carrying the antimicrobial agent.

In addition, the present disclosure relates to a coated expanded polytetrafluoroethylene (ePTFE) suture, comprises a base ePTFE suture comprising a surface on which a chemical etchant was applied, the chemical etchant comprising an alkali-based etchant to remove fluorine from the surface of the base ePTFE suture; and a coating applied to the base ePTFE suture, the coating comprising a cross linkable silicone polymer, the coating comprising an antimicrobial agent selected from the group including of triclosan, chlorhexidine, polyhexamethylene biguanide (PHMB), octenidine, and silver.

In an embodiment, the chemical etchant was applied by immersing the base PTFE suture in a container including the alkali-based etchant for a set duration at a set temperature until the surface of the ePTFE suture changes from a deep dark blue color to a transparent light brown color.

In an embodiment, the set duration comprises two minutes and the set temperature comprises 50°C, wherein applying the chemical etchant further comprises shaking the container periodically.

In an embodiment, the coating was applied by dipping the base ePTFE suture into a container including the coating, brushing, rolling, or spraying the coating onto the surface of the base ePTFE suture.

In an embodiment, the coating was cured by a convection oven with curing parameters including a set duration at a set temperature, or by flash curing.

In an embodiment, first curing parameters comprise 5 seconds at 150°C and second curing parameters comprise 120 seconds at 90°C.

In an embodiment, removing fluorine from the surface of the base ePTFE suture reduces a lubricity of the base ePTFE suture.

In an embodiment, the antimicrobial agent is released over a duration comprising at least 3 days.

In an embodiment, the alkali-based etchant comprises sodium naphthalene or sodium ammonia.

In an embodiment, curing the coating produces a cross-linked silicone matrix carrying the antimicrobial agent.

### Brief Description of the Drawings

Fig. 1 shows a method for fabricating a coated ePTFE suture according to various exemplary embodiments.
Fig. 2 shows a table demonstrating the antimicrobial stability of the coated ePTFE suture according to one example.

### Detailed Description

The exemplary embodiments may be further understood with reference to the following description and the appended drawings, wherein like elements are referred to with the same reference numerals. The exemplary embodiments relate to expanded polytetrafluoroethylene (ePTFE) sutures comprising an antimicrobial coating and methods for producing coated ePTFE sutures by a chemical etching process and a coating process.

Sutures can be formed of ePTFE as a monofilament, i.e., a strand of ePTFE. Sutures formed of ePTFE are biologically inert, non-absorbable, strong, extremely soft, and smooth, making ePTFE sutures ideal for a number of surgical procedures including, e.g., dental bone grafting and implant procedures, where a soft monofilament suture is desirable. ePTFE sutures have also shown excellent results for chordal replacement. ePTFE sutures are also easy to knot. However, there exists a risk of knots in ePTFE sutures unraveling due to the slippery nature of the ePTFE material. Postoperative knot failure can have dramatic consequences, requiring a repeat operation with cardiopulmonary bypass. Some surgeons compensate for this risk by securing the knot in more complicated ways, e.g., with multiple additional throws or by using a clip.

Another potential issue with ePTFE sutures is the porous nature of the material, which may permit bacterial and other pathogenic microorganisms to attach and penetrate the body of suture, aggravating infection.

Accordingly, there is a need to improve knot security of ePTFE sutures due to the slippiness of this type of material. More complicated knots can present challenges when the operation is performed endoscopically. Additionally, there is a need to improve the antimicrobial properties of ePTFE sutures. Accordingly, a solution is needed to address these two critical challenges with ePTFE sutures.

Coatings have been applied to sutures to improve suture performance by, e.g., reducing friction, improving knot security, reducing tissue reactivity, and/or providing antimicrobial properties. Common polymeric coatings include Polyglactin, Polycaprolactone and Polybutylate for improving handling and reducing friction. Antimicrobial protection ingredients (API) can be introduced into coatings to prevent bacterial colonization and reduce infection risks. Some common APIs include triclosan, chlorhexidine, polyhexamethylene biguanide (PHMB), octenidine, and silver.

Coatings can be applied to sutures by techniques including dipping, brushing, rolling, or spraying. Vapor deposition refers to a coating technique in which a solid/liquid coating material is evaporated at a set temperature, e.g., at a high temperature, and deposited in its gas phase onto a surface in controlled temperature/pressure conditions so that the vapor condenses and solidifies onto the surface.

ePTFE sutures are difficult to coat, which makes it difficult to incorporate triclosan into the suture. Vapor deposition can be utilized to deposit an API, e.g., triclosan, onto the suture surface, yet controlled release of the API from the suture is technically challenging. The loose affiliation of triclosan molecules on the surface of ePTFE leads to rapid release of this molecule. Antimicrobial sutures are preferably designed with controlled release so that their antimicrobial functions are maintained for a certain period, e.g., 3 days.

A silicone-based suture coating was developed including a cross-linkable silicone polymer, a silicone cross-linking agent, and an antimicrobial agent such as, e.g., triclosan, as described in commonly assigned U.S. Pat. No. 11,464,889, the disclosure of which is hereby incorporated by reference herein. The cross-linking process for the silicone polymer includes mild curing conditions such that the antimicrobial components remain in the cross-linked silicone matrix and do not decompose or evaporate. The antimicrobial agent can be homogenously distributed throughout the silicone matrix. This silicone-based antimicrobial coating has shown improvement on the knot performance of PET suture.

According to various exemplary embodiments, methods are described for improving the knot performance of ePTFE sutures while incorporating antimicrobial properties therein for an extended duration. The exemplary embodiments generally include a chemical etching process and a coating process. In general, a chemical etch is applied to the surface of an ePTFE suture to prepare the suture for coating by a coating agent such as, e.g., the silicone-based coating described above. In various embodiments described below, specific types of etching agents (i.e., etchants) and coatings are described in association with specific parameters for the etching and coating processes. Those skilled in the art will understand that the exemplary embodiments are not limited thereto and that different coatings may be used in accordance with the present embodiments, which could influence the selection of different etchants and associated modifications to the etching and coating processes described below.

In various embodiments, the ePTFE suture comprises a monofilament suture. Those skilled in the art will understand that the exemplary embodiments are not limited to monofilament sutures and that different types of ePTFE sutures, e.g., braided sutures, barbed sutures, etc., may be coated in accordance with the present embodiments. Additionally, the ePTFE suture can have different closure styles, e.g., standard (no pre-created closure for the thread), loop (a single loop at the end of the thread), tie (a preformed knot at the end of the thread that can be tightened), etc. It should be understood that the ePTFE suture according to the present embodiments shows improved knot security characteristics, such that a simpler closure style can generally be used without risk of a knot unraveling.

In some embodiments, a chemical etch is applied to the surface of an ePTFE suture. Since the lubricity of ePTFE is the result of its chemical structure, i.e., a fluorine sheath covering a chain of carbon atoms, an alteration of this chemical structure is necessary to attain true adhesion of a coating to the suture with a strong bond. The exemplary chemical etching process achieves this by bringing the ePTFE suture into contact with an etchant for altering the fluorine sheath, e.g., sodium naphthalene or sodium ammonia, by either dipping the suture into a tank or applying the etchant to the surface of the suture.

As these etchants are derived from sodium, an alkali metal, it chemically reacts with the ePTFE, removing the fluorine atoms from the ePTFE molecules. Those skilled in art will understand that alternative types of etchants may be suitable for altering the fluorine sheath of the ePTFE suture. Because the fluorine sheath is responsible for the lubricity of PTFE, removing most of the fluorine from the surface essentially removes this property, allowing the surface to be adhered to virtually any substrate, including silicone-based material.

In some embodiments, a coating is applied to the surface of an etched ePTFE suture. The control release characteristic of silicone makes it attractive to be used as a candidate carrier for ePTFE suture. In some embodiments, the coating comprises a silicone coating solution containing an antimicrobial agent, e.g., triclosan. Those skilled in the art will understand that alternative coating solutions may be suitable for coating the etched ePTFE suture.

The coated ePTFE sutures resulting from the disclosed chemical etching and coating techniques provide superior knot security and extended antimicrobial efficacy compared to the uncoated ePTFE suture. Prototypes of the coated ePTFE sutures underwent knot security testing and Zone of Inhibition (ZOI) testing to demonstrate the superior performance relative to uncoated ePTFE, to be described in greater detail below.

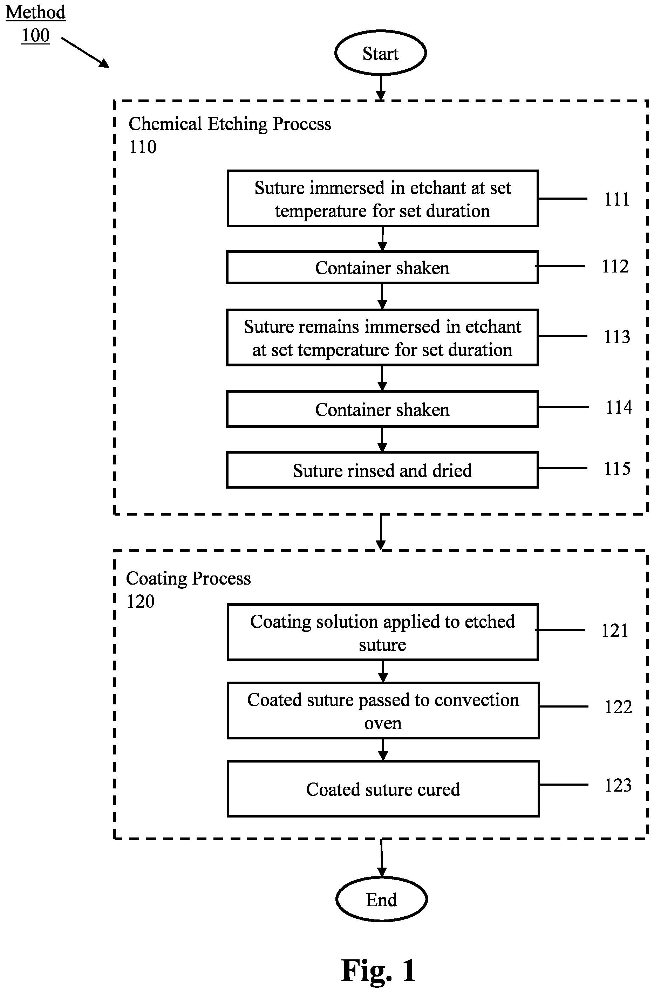

Fig. 1 shows a method 100 for fabricating a coated ePTFE suture according to various exemplary embodiments. The method 100 generally includes a chemical etching process 110, in which a chemical etch is applied to the ePTFE suture, and a coating process 120, in which a coating is applied to the etched suture. In some illustrative examples, the coating comprises a silicone-based coating and the chemical etchant comprises sodium naphthalene, as described in sub-steps 111-115 of the chemical etching process 110 and sub-steps 121-123 of the coating process 120 described below.

The method 100 relates to ePTFE sutures. In one embodiment, the ePTFE sutures comprise AeosTM USP 3, Zeus, Orangeburg, SC, 40 inch in length. Those skilled in art understand that any type of ePTFE sutures (currently commercially available or to be developed in the future) can be etched and coated according to the described embodiments. Any number of ePTFE sutures can be chemically etched and coated according to the present techniques, depending on the capacity of the equipment used for these processes.

In 110, the suture is immersed in etching solution (i.e., the etchant) in a container. In one embodiment, the etching solution comprises 40g FluroEtchTM Fluoropolymer pre-bonding etchant (Acton technology, Pittston, PA). As described above, in other embodiments, other etchants suitable for fluoropolymers may be used to remove the fluorine layer from the suture. The suture(s) can be immersed for a specific duration, and, in some cases, the container can be shaken.

In one example embodiment, in 111, the suture is immersed in the etchant at a set temperature for a set duration. In one example, 10 pieces of ePTFE sutures are fully immersed in the etching solution at 50degC for 1 minute. In 112, the etching solution is shaken, e.g., for 10 seconds. In 113, the suture remains immersed in the etching solution for another duration. In one example, the ePTFE sutures are fully immersed in the etching solution at 50degC for another 1 minute. In 114, the etching solution is shaken, e.g., for 10 seconds. The deep blue color of the etching solution turns to a very light-yellow color. The white ePTFE suture turns to a deep brown color as the result of this process. In 115, the etched sutures were rinsed with isopropanol and water. The etched sutures are then dried, e.g., at ambient conditions.

The chemical etching process 110 removes the fluorine from the surface of the ePTFE suture to reduce the lubricity of the suture. The resulting surface of the etched ePTFE suture is suitable for the application of a coating.

In 120, the etched sutures are coated. In some embodiments, the coating is applied by dipping. In other embodiments, the coating is applied by other conventional coating techniques such as brushing, rolling, or spraying. In some embodiments, the coating is a silicone-based coating containing triclosan, e.g., as described above with regard to U.S. Patent No. 11,464,889.

In one example embodiment, in 121, the silicone-based coating solution is applied to the surface of the etched ePTFE sutures by dip coating tanks. In 122, the sutures are passed to an in-line convection oven. In 123, the coating is cured by the oven at a set temperature for a set duration. In one example, the curing time can be 5 seconds at 150°C. In another example, the curing time can be 120 seconds at 90°C. The curing time/temperature is set to complete the vinyl silylation addition cross linking reaction of the silicone coating solution. Flash curing can also be used. The active antimicrobial component triclosan remains in the coating after the curing process due to its relatively low temperature and short process time.

In other embodiments, different types of coatings can be used, e.g., other silicone-based coatings or other polymer-based coatings. In these cases, different coating applications and curing parameters/techniques may be used.

The resulting coated suture shows enhanced knot security due to the decreased lubricity caused by the etchant reacting with the fluoride sheath of the original ePTFE suture. Although adding the silicone-based coating increases the lubricity of the coated ePTFE suture relative to the etched ePTFE suture, the lubricity of the coating is reduced relative to the original ePTFE suture such that the sutures remain sufficiently rough to enable a simple knot to hold for its intended duration without slipping.

Knot security testing was conducted to evaluate the behavior of a knot when the suture is under tension. The test is performed in a tensile tester at constant displacement. Ten specimens were tested for each group of suture samples. The suture was immersed in sodium chloride solution for 24 hours and tested wet. For each sample, the knot was tied around a plastic mandrel and 4 throws were tied for each strand of suture specimen. The failure mode, unraveling or breaking, was recorded for each specimen. The knot performance is rated as being secured (no slippage), slip and break, or slip through (no break).

The results of the knot security testing were all the knots from the prototype samples (triclosan containing silicone coated etched ePTFE samples) were secured, while all the knots from uncoated ePTFE sutures slipped through.

Testing of antimicrobial properties can comprise zone of inhibition (ZOI) testing. Zone of Inhibition testing is a conventional method for estimating the inhibitory effects of antimicrobial substances against specific bacterial strains of interest. ZOI assays are useful for testing diffusible agents. As the agent diffuses away from the disk, the concentration decreases logarithmically. The sensitivity of the organism to the agent is judged by the appearance and size of a Zone where no growth occurs, i.e., the Zone of Inhibition. Typically, a ZOI value of 3 mm is considered to be an effective zone to inhibit infection.

The prototype test articles were tested for antimicrobial properties utilizing this Zone of inhibition test. The test articles were aseptically placed in individual sterile Petri dishes and challenged with 100 micro liters of inoculum containing 10 colony-forming units (CFU) of Staphylococcus aureus or Escherichia coli. Trypticase soy agar (TSA) was poured into each dish and allowed to solidify. The plates were incubated at 37°C. for 48 hours. After incubation, the plates were examined under a darkfield colony counter, and the Zones of inhibition were measured.

The ZOI was measured in millimeters at three points along the suture: each cut end and the center of the strand. To evaluate the suture's ability to inhibit colonization over time, sutures were removed using sterile forceps and transferred to freshly inoculated plates daily (any residual TSA on the suture was removed aseptically). These plates were incubated overnight, the ZOIs measured, and the daily transfers continued until the third day, the results are summarized as follows.

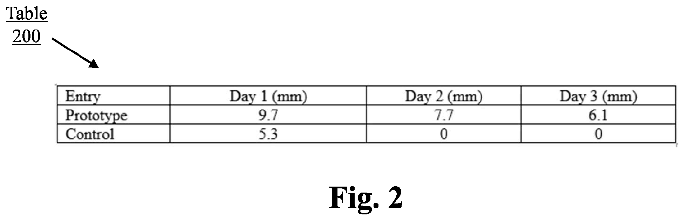

The control sutures comprise triclosan-coated ePTFE sutures prepared by conventional vapor deposition. Fig. 2 shows a table 200 demonstrating the antimicrobial stability of the coated ePTFE suture according to one example. As shown, the control sample maintained a ZOI of 5.3 mm after Day 1 and had no antimicrobial activity by Day 2. The prototype sample maintained a ZOI of 9.7 mm after Day 1, a ZOI of 7.7 mm after Day 2, and a ZOI of 6.1 mm after Day 3.

Accordingly, the triclosan containing silicone coated ePTFE suture exhibited antibacterial activity against E. coli in vitro. The activity was persistent for multiple days and was stable post sterilization. The activity of triclosan coated ePTFE suture prepared by conventional vapor deposition technique lasted only one day. The additional antimicrobial properties of the described embodiments would be a valuable adjunct in reducing the risk of SSI and the associated morbidity for patients and the financial impact on the healthcare systems.

It will be apparent to those skilled in the art that various modifications may be made in the present disclosure, without departing from the scope of the disclosure. Furthermore, those skilled in the art will understand that the features of any of the various embodiments may be combined in any manner that is not inconsistent with the description and/or the functionality of the embodiments.

## Claims

1. A method for coating an expanded polytetrafluoroethylene (ePTFE) suture, comprising:
applying a chemical etchant to a surface of the ePTFE suture, the chemical etchant comprising an alkali-based etchant to remove fluorine from the surface of the ePTFE suture to produce an etched ePTFE suture; and
applying a coating to the etched ePTFE suture to produce a coated ePTFE suture, the coating comprising a cross linkable silicone polymer, the coating comprising an antimicrobial agent selected from the group including of triclosan, chlorhexidine, polyhexamethylene biguanide (PHMB), octenidine, and silver.

2. The method of claim 1, wherein applying the chemical etchant comprises:
immersing the PTFE suture in a container including the alkali-based etchant for a set duration at a set temperature until the surface of the PTFE suture changes from a deep dark blue color to a transparent light brown color, optionally wherein the set duration comprises two minutes and the set temperature comprises 50°C, optionally wherein applying the chemical etchant further comprises shaking the container periodically.

3. The method of claim 1 or claim 2, wherein applying the coating comprises:
dipping the etched ePTFE suture into a container including the coating; or
brushing, rolling, or spraying the coating onto the surface of the etched ePTFE suture.

4. The method of any preceding claim, further comprising:
curing the coating by a convection oven with curing parameters including a set duration at a set temperature, or
flash curing the coating;
optionally wherein first curing parameters comprise 5 seconds at 150°C and second curing parameters comprise 120 seconds at 90°C.

5. The method of any preceding claim, wherein removing fluorine from the surface of the ePTFE suture reduces a lubricity of the etched ePTFE suture relative to the ePTFE suture, wherein a lubricity of the coated ePTFE suture is reduced relative to the ePTFE suture.

6. The method of any preceding claim, wherein the coated ePTFE suture comprises a controlled release of the antimicrobial agent over a duration comprising at least 3 days.

7. The method of any preceding claim, wherein the alkali-based etchant comprises sodium naphthalene or sodium ammonia.

8. The method of any preceding claim, wherein curing the coating produces a cross-linked silicone matrix carrying the antimicrobial agent.

9. A coated expanded polytetrafluoroethylene (ePTFE) suture, comprising:
a base ePTFE suture comprising a surface on which a chemical etchant was applied, the chemical etchant comprising an alkali-based etchant to remove fluorine from the surface of the base ePTFE suture; and
a coating applied to the base ePTFE suture, the coating comprising a cross linkable silicone polymer, the coating comprising an antimicrobial agent selected from the group including of triclosan, chlorhexidine, polyhexamethylene biguanide (PHMB), octenidine, and silver.

10. The coated ePTFE suture of claim 9, wherein the chemical etchant was applied by immersing the base PTFE suture in a container including the alkali-based etchant for a set duration at a set temperature until the surface of the ePTFE suture changes from a deep dark blue color to a transparent light brown color, optionally wherein the set duration comprises two minutes and the set temperature comprises 50°C, wherein applying the chemical etchant further comprises shaking the container periodically.

11. The coated ePTFE suture of claim 10, wherein the coating was applied by dipping the base ePTFE suture into a container including the coating, or brushing, rolling, or spraying the coating onto the surface of the base ePTFE suture.

12. The coated ePTFE suture of claim 11, wherein the coating was cured by a convection oven with curing parameters including a set duration at a set temperature, or by flash curing, optionally wherein first curing parameters comprise 5 seconds at 150°C and second curing parameters comprise 120 seconds at 90°C.

13. The coated ePTFE suture of any one of claims 9 to 12, wherein removing fluorine from the surface of the base ePTFE suture reduces a lubricity of the base ePTFE suture.

14. The coated ePTFE suture of any one of claims 9 to 13, wherein the antimicrobial agent is released over a duration comprising at least 3 days.

15. The coated ePTFE suture of any one of claims 9 to 14, wherein the alkali-based etchant comprises sodium naphthalene or sodium ammonia.

16. The coated ePTFE suture of any one of claims 9 to 15, wherein curing the coating produces a cross-linked silicone matrix carrying the antimicrobial agent.
